# FASCICULE DE BREVET EUROPEEN

(11) **EP 3 525 900 B1**
(45) Date de publication et mention de la délivrance du brevet: **23.09.2020**
(21) Numéro de dépôt: 17780782.3
(22) Date de dépôt: 13.10.2017
(51) Int. Cl.: C07C 29/88, C07C 45/80, B01D 11/04, B01D 11/00

(54) **PROCEDE DE PURIFICATION D'UNE SOLUTION AQUEUSE COMPRENANT DU DIETHYLACETAL**
VERFAHREN ZUR REINIGUNG EINER WÄSSRIGEN LÖSUNG UMFASSEND DIETHYLACETAL
PROCESS FOR PURIFYING AN AQUEOUS SOLUTION COMPRISING DIETHYLACETAL

(30) Priorité: 17.10.2016 FR 1660015
(43) Date de publication de la demande: 21.08.2019
(73) Titulaire: IFP Energies nouvelles, 92852 Rueil-Malmaison (FR); Compagnie Générale des Etablissements Michelin, 63000 Clermont-Ferrand (FR)
(72) Inventeur: HOGNON, Céline, 69780 Mions (FR); DROZDZ, Sophie, 69126 Brindas (FR); JACQUIN, Marc, 69002 Lyon (FR); LEINEKUGEL LE COCQ, Damien, 69007 Lyon (FR); AUGIER, Frederic, 69360 Saint Symphorien d'Ozon (FR)
(74) Mandataire: IFP Energies nouvelles
(86) Numéro de dépôt international: PCT/EP2017/076229
(87) Numéro de publication internationale: WO 2018/073129

(56) Documents cités:
- FR-A1- 3 026 100
- FR-A1- 3 026 101

## Description

### DOMAINE TECHNIQUE DE L'INVENTION

La présente invention concerne l'amélioration de la récupération de l'éthanol et de l'acétaldéhyde dans les effluents liquides de réacteurs Lebedev.

### ART ANTERIEUR

Le procédé de production de butadiène à partir d'éthanol a été développé, en particulier, par des équipes américaines durant la seconde guerre mondiale à partir des travaux d'Ostromilenski.

Dans ce procédé, la conversion par passe est inférieure à 50%, ce qui implique des recyclages importants de l'éthanol et de l'acétaldéhyde. De plus, une grande variété d'impuretés de différentes natures (hydrocarbures saturés, insaturés, aromatiques, produits oxygénés tels que alcools, cétones, aldéhydes, phénols, acides, esters, éthers) et ayant des masses molaires très différentes est produite (entre 50 et 10 000 g/mol).

Il est donc nécessaire de mettre en place un enchaînement d'opérations unitaires dans le but d'éliminer le maximum d'impuretés en perdant le moins possible d'éthanol et d'acétaldéhyde. D'un point de vue économique, il est primordial de diminuer le coût de production du butadiène, ce qui nécessite de :
- perdre le moins possible d'éthanol et d'acétaldéhyde
- ne pas recycler d'impuretés dans les réacteurs qui induiraient une chute de sélectivité en butadiène, ou qui s'accumuleraient à des niveaux non acceptables, nécessitant une purge et donc des pertes en éthanol et acétaldéhyde.

À la sortie des réacteurs catalytiques, l'effluent produit composé de butadiène, d'acétaldéhyde, d'eau, d'éthanol et d'impuretés subit plusieurs opérations unitaires afin de séparer les sous-produits gazeux des sous-produits liquides, liquides et gazeux étant entendus à température et pression ambiante.

Parmi les sous-produits gazeux, on peut citer l'hydrogène, le monoxyde de carbone, le dioxyde de carbone, les alcanes et les oléfines en C₁-C₄. Il est primordial d'éliminer ces sous-produits de l'effluent riche en butadiène afin d'obtenir un produit aux spécifications.

Parmi les sous-produits liquides à température ambiante, on peut citer l'acétone, le diéthyléther, le butanal, le butanol, le butanone, l'éthyle acétate, le crotonaldéhyde et l'acide acétique. D'autres sous-produits sont générés en plus faible quantité dans la zone réactionnelle. Dans la suite du document, on désignera par « impuretés » cet ensemble de milliers de composés hydrocarbonés ou oxygénés.

Dans les premiers schémas de procédé des équipes américaines, l'éthanol, l'acétaldéhyde, l'eau et les sous-produits liquides étaient séparés par un enchaînement de trois colonnes à distiller (brevet US 2,403,742). L'effluent riche en éthanol, acétaldéhyde, eau et sous-produits liquides alimente une première colonne à distiller dans laquelle un effluent riche en acétaldéhyde est séparé du reste de l'effluent. Une seconde colonne à distiller permet de séparer les sous-produits liquides d'un effluent riche en éthanol et eau. La dernière colonne à distiller permet de séparer l'éthanol de l'eau. La majeure partie des brevets de procédé déposés dans la période 1940-1960 par les sociétés Carbide & Carbon ou Koppers (US 2,403,743 ; US 2,393,381 ; US 2,395,057 et US 2,439,587) visent à améliorer cette partie du schéma.

Une des problématiques du procédé, observée dans les années 1945, est une formation importante de diéthylacétal entrainant une perte non négligeable en réactifs et donc en rendement en butadiène. Toussaint et al., Industrial and Engineering Chemistry; 1947; Vol. 39, No. 2, p. 120-125, indiquent que 20 kg de diéthylacétal sont produits pour une tonne de butadiène formé.

Dans les brevets FR 3 026 100 et FR 3 026 101, l'élimination des impuretés liquides se fait par extraction liquide-liquide. L'effluent composé d'éthanol, d'acétaldéhyde, d'eau et d'impuretés alimente une colonne d'extraction liquide-liquide. Cette dernière est alimentée en fond par un solvant de lavage qui a pour but de laver à contre-courant la charge. A la sortie de cette section de lavage, l'extrait est composé majoritairement du solvant de lavage, des sous-produits extraits et d'une faible quantité d'éthanol et d'acétaldéhyde. Cet extrait est ensuite lavé à l'eau dans le but de ré-extraire l'éthanol et l'acétaldéhyde et ainsi minimiser les pertes en éthanol et acétaldéhyde. Le solvant de lavage employé pour cette opération unitaire est constitué d'un mélange d'hydrocarbures ayant entre 6 et 40 atomes de carbones.

Dans cette configuration, une forte proportion du diéthylacétal et environ la moitié de l'hémi-acétal correspondant, formés en amont de l'étape d'extraction liquide-liquide, sont extraits par le solvant. Cela a pour conséquence d'entrainer une perte en équivalent éthanol et acétaldéhyde non négligeable et donc augmenter le prix de production du butadiène. Un objet de l'invention est d'améliorer la récupération de l'éthanol et de l'acétaldéhyde et s'applique avantageusement à l'effluent éthanol / acétaldéhyde / eau issu de D1) de FR 3 026 100, ou l'effluent éthanol/acétaldéhyde/eau issu de l'étape B) de FR 3 026 101.

### OBJET ET INTERET DE L'INVENTION

L'invention concerne un enchainement d'opérations unitaires pour l'extraction des impuretés et la décomposition du diéthylacétal contenue dans une charge de type Lebedev composé d'eau, d'éthanol, d'acétaldéhyde et d'impuretés.

La demanderesse a découvert qu'en contrôlant un certain nombre de paramètres opératoires telles que le pH de la solution aqueuse utilisée pour réaliser l'étape de réextraction et la température de l'extraction liquide-liquide, on pouvait de manière surprenante limiter les pertes en éthanol et acétaldéhyde lors de l'extraction du diethylacetal et de l'hémiacetal. L'emploi d'eau acidifiée pour réaliser la contre-extraction de l'éthanol et de l'acétaldéhyde, l'augmentation de la température et du temps de séjour dans l'extracteur peuvent, dans des conditions particulières, améliorer les performances du procédé de production de butadiène à partir d'éthanol.

### DESCRIPTION DETAILLEE DE L'INVENTION

L'invention concerne un procédé de purification d'une solution aqueuse comprenant au moins de l'éthanol, de l'acétaldéhyde et du diéthylacétal comprenant :
- une étape A) d'extraction liquide-liquide à contre-courant comprenant une section d'extraction alimentée en tête par ladite solution aqueuse en mélange avec au moins une fraction du raffinat eau/éthanol/acétaldéhyde issu de l'étape B) de réextraction, ce mélange constituant la charge de ladite section d'extraction de ladite étape A), et en fond par un solvant d'extraction, et produisant en tête un extrait et en fond une charge purifiée, opérée à une température comprise entre 10 et 40°C et à une pression comprise entre 0,1 et 0,5 MPa avec un rapport de débit massique de phase continue / débit massique de phase dispersée inférieur à 70 ;
- une étape B) de réextraction liquide-liquide à contre-courant alimentée en tête par une solution aqueuse acide dont le pH est compris entre 0,5 et 5, et en fond par l'extrait issu de l'étape A), et produisant en tête un extrait et en fond un raffinat eau/éthanol/acétaldéhyde, opérée à une température comprise entre 10 et 90°C et à une pression comprise entre 0,1 et 0,5 MPa avec un avec un rapport de débit massique de phase continue / débit massique de phase dispersée inférieur à 70, de préférence inférieure à 35, de manière préférée inférieure à 10, et de préférence inférieur à 3.

### Charge du procédé

Le procédé selon l'invention permet d'extraire l'éthanol et l'acétaldéhyde d'une solution aqueuse comprenant de l'éthanol, de l'acétaldéhyde, du diéthylacétal et des impuretés. Les impuretés peuvent être de natures très variées (hydrocarbures saturés, insaturés, aromatiques, produits oxygénés, parmi lesquels on peut citer les alcools, cétones, aldéhydes, composés phénoliques, acides, esters, éthers), la masse molaire des différentes impuretés pouvant aller de 50 à 10 000 g/mol. Parmi les impuretés typiques, on peut citer l'acétone, le diéthyléther, le butanal, des butanols, des butanones, l'éthyle acétate, le crotonadélhyde, des pentènes, des pentadiènes, des hexènes et des hexadiènes.

Ledit procédé de purification est alimenté par une solution aqueuse comprenant au moins de l'éthanol, de l'acétaldéhyde et du diéthylacétal.

De préférence, la teneur en éthanol dans ladite solution aqueuse est comprise entre 40 % et 70% poids, de préférence entre 50 et 60% poids, la teneur en acétaldéhyde est comprise entre 1 et 30% poids, de préférence entre 5 et 10% poids et la teneur en impuretés est comprise entre 1 et 20% poids, de préférence entre 5 et 20% poids. La teneur en diéthylacétal dans ladite solution aqueuse est de préférence comprise entre 1 et 20% poids, et de manière préférée entre 1 et 15% poids. Ladite solution aqueuse est avantageusement un effluent de conversion d'éthanol, ou d'un mélange éthanol/acétaldéhyde en 1,3-butadiène après séparation des incondensables et du butadiène. Cette solution aqueuse est de manière préférée un effluent semblable à l'effluent éthanol / acétaldéhyde / eau issu de D1) de FR 3 026 100, ou l'effluent éthanol/acétaldéhyde/eau issu de l'étape B) de FR 3 026 101 ou tout autre effluent équivalent de procédé de type Lebedev.

### Étape A) d'extraction des impuretés

Le procédé de purification selon l'invention comprend une étape A) d'extraction liquide-liquide à contre-courant comprenant une section d'extraction alimentée en tête par ladite solution aqueuse en mélange avec le raffinat issu de l'étape B) de réextraction, ce mélange constituant la charge de ladite section d'extraction, et en fond par un solvant d'extraction, et produisant en tête un extrait et en fond une charge purifiée, opérée à une température comprise entre 10 et 70°C et à une pression comprise entre 0,1 et 0,5 MPa, préférentiellement entre 0,2 et 0,4 MPa, avec un rapport de débit massique de phase continue / débit massique de phase dispersée inférieur à 70, de préférence inférieure à 35, de manière préférée inférieure à 10, et de préférence inférieur à 3, de préférence inférieur à 1,5. Au-delà de 70, le fonctionnement hydrodynamique de la section d'extraction est compromis. Peu importe que le solvant d'extraction forme la phase continue ou dispersée, ce critère étant un critère hydrodynamique.

Le temps de séjour dans la section d'extraction de ladite étape A) est ajusté de manière à obtenir les performances désirées en terme de taux de récupération, comme il est connu de l'Homme du métier. Il est typiquement compris entre 0,5 et 10 h, avantageusement entre 0,5 et 6 h.

Comme connu de l'Homme du métier, une extraction liquide-liquide fonctionne avec deux phases liquides, l'une des phases constituant la phase continue et l'autre constituant la phase dispersée, présente sous forme de gouttes distinctes. La nature continue ou dispersée dépend du débit relatif d'une phase par rapport à l'autre. Ainsi, selon le phénomène bien connu, si l'on réduit le débit de la phase continue en augmentant le débit de la phase dispersée, la phase dispersée deviendra continue et inversement.

Ledit extrait produit en tête de ladite section d'extraction de ladite étape A) comprend de préférence plus de 80% poids des impuretés, entre 20 et 50% poids de l'éthanol et de l'acétaldéhyde et plus de 90% du diéthylacétal contenus dans ladite charge de ladite section d'extraction de ladite étape A).

Ladite charge purifiée produite en fond de ladite section d'extraction de ladite étape A) comprend de l'eau et entre 80 et 100%poids de l'éthanol et de l'acétaldéhyde contenus dans ladite solution aqueuse alimentant le procédé selon l'invention. En d'autres termes, entre 80 et 100% de l'éthanol et de l'acétaldéhyde alimentant le procédé selon l'invention sont récupérés dans ladite charge purifiée.

Le solvant d'extraction et la solution aqueuse alimentant ladite étape A) sont alimentés chacun à une température indépendamment comprise entre 10 et 70°C, préférentiellement entre 20 et 55°C.

Plus le rapport débit massique de solvant d'extraction / débit massique de l'alimentation de ladite section d'extraction de ladite étape A) est élevé, plus l'étape d'extraction des impuretés est efficace. Cependant, un ratio élevé conduit à extraire également une fraction importante d'éthanol et d'acétaldéhyde dans l'extrait de ladite étape A), et par conséquent à augmenter le débit de solution aqueuse acide nécessaire au sein de l'étape B) pour limiter les pertes en éthanol et acétaldéhyde. La valeur du ratio de débit massique de solvant d'extraction sur le débit massique de solution aqueuse acide doit donc être ajusté de manière à extraire le maximum d'impuretés tout en limitant les pertes en éthanol et acétaldéhyde.

Le ratio de débit massique de solvant d'extraction sur le débit massique de solution aqueuse acide est ajusté de manière à ce que ledit extrait issu de l'étape B) comprenne 50% poids, de préférence 60% poids et de manière préférée 70% poids des impuretés contenues dans ladite solution aqueuse alimentant ladite étape A), ainsi qu'au plus 5% poids, de préférence au plus 2% poids, et de manière préférée au plus 1% poids de la quantité totale d'éthanol et d'acétaldéhyde contenue dans ladite solution aqueuse alimentant ladite étape A).

Ledit solvant d'extraction qui alimente l'étape A) est avantageusement un mélange d'hydrocarbures ayant entre 6 et 40 atomes de carbones, de préférence entre 10 et 20 atomes de carbone ou tout autre solvants permettant une démixtion avec la phase hydro-alcoolique. De manière non limitative, ledit mélange d'hydrocarbures peut être une coupe gazole ou kérosène désulfurée ou bien encore une coupe d'hydrocarbures produite par une unité de type Fischer-Tropsh.

Cette étape est configurée de manière à extraire le maximum d'impuretés et le minimum d'éthanol et d'acétaldéhyde. Dans une telle configuration, le diéthylacétal étant beaucoup moins polaire que l'éthanol et l'acétaldéhyde, il est extrait de manière quantitative avec les impuretés.

Un point important à noter est que, au sein de ladite charge alimentant l'étape A), la concentration en diéthylacétal est inférieure à l'équilibre thermodynamique de la réaction de conversion de l'éthanol et de l'acétaldéhyde en diéthylacétal. En effet, ladite charge provient d'un réacteur catalytique fonctionnant à haute température (température où le diéthylacétal est quantitativement décomposé), puis subit un certain nombre d'opération unitaires de séparation à une température modérée (favorable à la formation de diéthylacétal), mais a priori avec un temps de séjour trop faible pour que la quantité de diéthylacétal formée atteigne l'équilibre thermodynamique.

Le contact entre les deux phases liquides dans ladite section d'extraction est réalisé au sein d'un contacteur liquide-liquide. Différents modes de contact peuvent être envisagés. On peut citer de manière non limitative, une colonne garnie, une colonne pulsée, une colonne compartimentée agitée ou bien une batterie de mélangeur-décanteur. Ledit extrait de ladite étape A) alimente l'étape B) de ré-extraction de l'éthanol et de l'acétaldéhyde.

### Etape B) de ré-extraction de l'éthanol et de l'acétaldéhyde

Le procédé de purification selon l'invention comprend une étape B) de réextraction liquide-liquide à contre-courant comprenant une section de réextraction alimentée en tête par une solution aqueuse acide ayant un pH compris entre 0,5 et 5, et en fond par l'extrait issu de l'étape A), et produisant en tête un extrait et en fond un raffinat eau/éthanol/acétaldéhyde, opérée à une température comprise entre 10 et 70°C, préférentiellement entre 20 et 55°C, et à une pression comprise entre 0,1 et 0,5 MPa, préférentiellement entre 0,2 et 0,4 MPa, un temps de séjour compris entre 0,5 et 6 h, avantageusement entre 1 et 3 h, avec un rapport de débit massique de phase continue / débit massique de phase dispersée inférieur à 70, de préférence inférieure à 35, de manière préférée inférieure à 10, et de préférence inférieur à 3, de préférence inférieur à 1,5, au-delà de 70, le fonctionnement hydrodynamique de la section de réextraction étant compromis.

Le temps de séjour est défini comme le temps moyen nécessaire à une molécule d'eau injectée avec la solution aqueuse acide alimentant ladite section de réextraction de ladite étape B) pour être extraite dans le raffinat eau/éthanol/acétaldéhyde issu de ladite section de réextraction de ladite étape B). Ce temps de séjour est classiquement déterminée par mesure de DTS, ou Distribution de Temps de Séjour, dans laquelle un marqueur (colorant ou autre), est injecté ponctuellement en entrée, la concentration en ce marqueur étant observée en sortie.

Ladite solution aqueuse acide qui alimente l'étape B) est de l'eau acidifiée c'est-à-dire une eau ayant un pH compris entre 0,5 et 5 de préférence entre 2 et 4, et de manière préférée entre 2,5 et 3,5, et contient avantageusement moins de 2% poids du total (éthanol+acétaldéhyde), c'est-à-dire que la somme des teneurs massiques en éthanol et acétaldéhyde est inférieure à 2% poids de ladite solution aqueuse, de manière préférée moins de 1% poids et de manière très préférée ne contenant ni d'éthanol, ni acétaldéhyde. L'eau peut contenir de manière non limitative des acides forts et/ou des acides faibles. De manière non limitative, on peut employer pour acidifier l'eau un acide faible comme de l'acide acétique, ou un acide fort comme l'acide sulfurique ou l'acide nitrique.

La demanderesse a observé que des performances particulièrement intéressantes étaient obtenues si le temps de séjour t dans ladite section de réextraction de ladite étape B) de réextraction exprimé en heure et le pH de la solution aqueuse acide alimentant ladite étape B) étaient réglés de façon conjointe de sorte que pH-log₁₀(t/t₀) est compris entre 1 et 4, de manière préférée entre 1,5 et 3, et de manière avantageuse entre 1,8 et 2,5, t₀ représentant un temps de référence égal à 1 h. Par log₁₀(x), on entend le logarithme en base 10 de x, égal à ln(x)/ln(10).

Ladite solution aqueuse acide et ledit extrait issu de l'étape A) sont alimentés indépendamment à une température comprise entre 10 et 70°C, préférentiellement entre 20 et 55°C.

L'extrait issu de l'étape B) peut être traité dans une étape de séparation afin de récupérer et de recycler le solvant d'extraction vers l'étape A) d'extraction.

Au moins une fraction du raffinat produit en fond de ladite section de réextraction de ladite étape B), avantageusement la totalité dudit raffinat, est mélangé à la solution aqueuse alimentant ladite étape A). Même lorsque la totalité dudit raffinat est recyclé et comme il est de pratique courante en cas de recyclage, un soutirage peut être réalisé de manière continue ou discontinue sur ledit raffinat, appelé purge, afin de limiter l'accumulation des impuretés dans ce raffinat.

Le contact entre les deux phases liquides dans ladite section de réextraction de ladite étape B) de réextraction est avantageusement réalisé au sein d'un contacteur liquide-liquide. Différents modes de contact peuvent être envisagés. On peut citer de manière non limitative, une colonne à garnissage, une colonne pulsée, une colonne compartimentée agitée ou bien une batterie de mélangeur-décanteur.

Pour l'homme du métier, plus la température est élevée, plus le pH est bas et plus le temps de séjour est élevé au sein des opérations unitaires de séparation, plus nous devrions former de diéthylacétal, car cela conduit à augmenter la cinétique de réaction tout en laissant à celle-ci d'avantage de temps pour se dérouler, ce qui conduit à une augmentation des pertes en éthanol et en acétaldéhyde et augmente la teneur en diéthylacétal dans l'extrait. Or le procédé selon l'invention met en œuvre un pH faible dans l'étape de réextraction et un temps de séjour relativement élevé, tout en aboutissant à une meilleure récupération de l'éthanol et de l'acétaldéhyde.

De manière surprenante, la demanderesse a découvert qu'en contrôlant un certain nombre de paramètres opératoires telles que le pH de l'eau acidifiée, la température de la section de réextraction de l'éthanol et de l'acétaldéhyde et/ ou en modifiant le mode de contactage (temps de séjour et technologie), on pouvait de manière surprenante limiter les pertes en éthanol et acétaldéhyde.

De manière avantageuse, l'étape B) sera opérée de telle sorte que la phase aqueuse constitue la phase continue de ladite section de réextraction de ladite étape B) tandis que l'étape A) sera opérée de telle sorte que la phase organique constitue la phase continue de ladite section d'extraction de ladite étape A).

### DESCRIPTION DES FIGURES

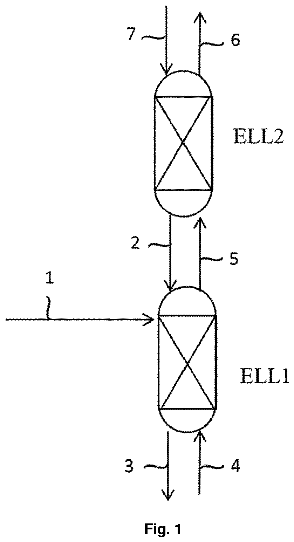

La **fig.1** représente de manière schématique et non limitative un arrangement du procédé selon l'invention.

La solution aqueuse comprenant de l'éthanol, de l'acétaldéhyde et du diéthylacétal (1) alimente en tête une colonne d'extraction liquide-liquide ELL1 dans laquelle est mise en œuvre l'étape A). Cette dernière est alimentée en tête par un raffinat eau/éthanol/acétaldéhyde (2) issu de la colonne d'extraction liquide-liquide ELL2 dans laquelle est mise en œuvre l'étape B), et en fond par le solvant d'extraction (4). L'extrait (5) est soutiré en tête de la colonne tandis qu'une charge purifiée (3) est soutirée en fond de la colonne.

L'extrait (5) alimente en fond la seconde colonne d'extraction liquide-liquide ELL2, qui est également alimentée en tête par une solution aqueuse acide (7). L'extrait (6) est soutiré en tête tandis qu'en fond de la colonne un raffinat eau/éthanol/acétaldéhyde (2) est soutiré et alimente la première colonne d'extraction liquide-liquide ELL1.

### EXEMPLES

Dans tous les exemples suivant, une solution aqueuse comprenant de l'éthanol, de l'acétaldéhyde et de l'eau présentant la composition suivante est traitée :
- 48% poids d'éthanol
- 9% poids d'acétaldéhyde
- 42% poids d'eau
- 1% poids de diéthyléther

### Exemple 1 (non-conforme)

*Dans cet exemple, l'étape de réextraction est mise en œuvre avec de l'eau pure.*

### Etape A) d'extraction liquide-liquide

La solution aqueuse comprenant de l'éthanol, de l'acétaldéhyde et de l'eau est injectée en tête d'une colonne agitée d'extraction liquide-liquide pilote de type Kuhni (ECR) de 1,8 m de hauteur utile et 32 mm de diamètre interne, munie d'un arbre d'agitation avec mobiles d'agitation et couronnes d'ouverture 40%. De l'hexadécane est injecté en fond en tant que solvant d'extraction. Les conditions d'opération sont les suivantes :
- Température : 23°C
- Phase organique continue, phase aqueuse dispersée
- Débit de charge : 4,8 L/h
- Débit de solvant : 5,2 L/h
- Vitesse d'agitation : 200 rotations par minute

### Etape B) de réextraction

L'extrait soutiré en tête de la colonne d'extraction est alimenté en fond d'une colonne de réextraction. La colonne de réextraction est identique à la colonne d'extraction (étape A). Cette colonne est alimentée en tête avec de l'eau pure, la phase aqueuse constituant la phase dispersée. Le raffinat soutiré en fond de colonne est mélangé à la solution aqueuse alimentant l'étape A).

Les performances suivantes sont obtenues :
- Le rendement en éthanol, défini comme le débit d'éthanol récupérée dans la charge purifiée soutirée en fond de la colonne de l'étape A) sur le débit d'éthanol dans la solution aqueuse alimentant l'étape A), est de 96%.
- Le rendement en acétaldéhyde, défini comme le débit d'acétaldéhyde récupérée dans la charge purifiée soutirée en fond de la colonne de l'étape A) sur le débit d'acétaldéhyde dans la solution aqueuse alimentant l'étape A), est de 91%.

### Exemple 2 (conforme)

*Dans cet exemple, l'étape B) de réextraction est mise en œuvre avec une solution aqueuse acide, la phase organique constituant la phase continue de l'étape B).*

L'étape A) d'extraction est réalisée de manière identique à l'exemple 1.

L'extrait soutiré en tête de la colonne d'extraction est alimenté en fond d'une colonne de réextraction. La colonne de réextraction est identique à la colonne d'extraction (étape A). Cette colonne est alimentée en tête avec une solution aqueuse contenant 3% poids d'acide acétique (soit un pH d'environ 2,5), la phase aqueuse constituant la phase dispersée.

En réalisant la réextraction avec de l'eau acidifiée comme phase dispersée, on obtient les performances suivantes :
- Le rendement en éthanol, défini comme le débit d'éthanol récupérée dans la charge purifiée soutirée en fond de la colonne de l'étape A) sur le débit d'éthanol dans la solution aqueuse alimentant l'étape A), est de 97%.
- Le rendement en acétaldéhyde, défini comme le débit d'acétaldéhyde récupérée dans la charge purifiée soutirée en fond de la colonne de l'étape A) sur le débit d'acétaldéhyde dans la solution aqueuse alimentant l'étape A), est de 92,5%.

Les performances sont améliorées par rapport à l'art antérieur dans lequel de l'eau pure est utilisée comme solvant de réextraction.

### Exemple 3 (conforme)

*Dans cet exemple, l'étape B) de réextraction est mise en œuvre avec une solution aqueuse acide, la phase aqueuse constituant la phase continue de l'étape B).*

L'étape A) d'extraction est réalisée de manière identique aux exemples 1 et 2.

L'extrait soutiré en tête de la colonne d'extraction est alimenté en fond d'une colonne de réextraction. La colonne de réextraction est identique à la colonne d'extraction (étape A). Cette colonne est alimentée en tête avec une solution aqueuse contenant 3% poids d'acide acétique (soit un pH d'environ 2,5), la phase aqueuse constituant la phase dispersée.

En réalisant la réextraction avec de l'eau acidifiée comme phase continue, on obtient les performances suivantes :
- Le rendement en éthanol, défini comme le débit d'éthanol récupérée dans la charge purifiée soutirée en fond de la colonne de l'étape A) sur le débit d'éthanol dans la solution aqueuse alimentant l'étape A), est supérieur à 99,5%.
- Le rendement en acétaldéhyde, défini comme le débit d'acétaldéhyde récupérée dans la charge purifiée soutirée en fond de la colonne de l'étape A) sur le débit d'acétaldéhyde dans la solution aqueuse alimentant l'étape A), est de 99,2%.

Les performances sont améliorées par rapport à une mise en œuvre phase organique continue dans l'étape A) / phase organique continue dans l'étape B).

## Revendications

1. Procédé de purification d'une solution aqueuse comprenant au moins de l'éthanol, de l'acétaldéhyde et du diéthylacétal comprenant :
- une étape A) d'extraction liquide-liquide à contre-courant comprenant une section d'extraction alimentée en tête par ladite solution aqueuse en mélange avec au moins une fraction du raffinat eau/éthanol/acétaldéhyde issu de l'étape B) de réextraction, ce mélange constituant la charge de ladite section d'extraction de ladite étape A), et en fond par un solvant d'extraction, et produisant en tête un extrait et en fond une charge purifiée, opérée à une température comprise entre 10 et 40°C et à une pression comprise entre 0,1 et 0,5 MPa avec un rapport de débit massique de phase continue / débit massique de phase dispersée inférieur à 70 ;
- une étape B) de réextraction liquide-liquide à contre-courant comprenant une section de réextraction alimentée en tête par une solution aqueuse acide dont le pH est compris entre 0,5 et 5, et en fond par l'extrait issu de l'étape A), et produisant en tête un extrait et en fond un raffinat eau/éthanol/acétaldéhyde, opérée à une température comprise entre 10 et 90°C et à une pression comprise entre 0,1 et 0,5 MPa, un temps de séjour compris entre 0,5 et 6 h, avec un avec un rapport de débit massique de phase continue / débit massique de phase dispersée inférieur à 70.

2. Procédé selon la revendication 1 dans lequel la teneur en éthanol dans ladite solution aqueuse comprenant de l'éthanol, de l'acétaldéhyde et du diéthylacétal est comprise entre 40 % et 70% poids, la teneur en acétaldéhyde est comprise entre 1 et 30% poids, la teneur en impuretés est comprise entre 1 et 20% poids, et la teneur en diéthylacétal est comprise entre 1 et 20% poids.

3. Procédé selon l'une des revendications précédentes dans lequel ledit solvant d'extraction qui alimente l'étape A) est un mélange d'hydrocarbures ayant entre 6 et 40 atomes de carbones.

4. Procédé selon l'une des revendications précédentes dans lequel le rapport de débit massique de phase continue / débit massique de phase dispersée dans ladite section d'extraction de ladite étape A) est inférieur à 3.

5. Procédé selon l'une des revendications précédentes dans lequel ladite solution aqueuse acide qui alimente l'étape B) a un pH compris entre 2 et 4.

6. Procédé selon l'une des revendications précédentes dans lequel le temps de séjour *t* dans ladite section de réextraction de ladite étape B) de réextraction exprimé en heure et le pH de la solution aqueuse acide alimentant ladite étape B) sont réglés de façon conjointe de sorte que pH-log₁₀(t/t₀) est compris entre 1 et 4, t₀ représentant un temps de référence égal à 1 h.

7. Procédé selon l'une des revendications précédentes dans lequel ladite solution aqueuse acide qui alimente l'étape B) contient moins de 2% poids du total (éthanol+acétaldéhyde).

8. Procédé selon l'une des revendications précédentes dans lequel ladite solution aqueuse acide qui alimente l'étape B) contient moins de 1% poids du total (éthanol+acétaldéhyde).

9. Procédé selon l'une des revendications précédentes dans lequel ladite solution aqueuse acide qui alimente l'étape B) ne contient ni éthanol, ni acétaldéhyde.

10. Procédé selon l'une des revendications précédentes dans lequel la phase organique constitue la phase continue de ladite section d'extraction de ladite étape A) et la phase aqueuse constitue la phase continue de ladite section de réextraction de ladite étape B).

## Patentansprüche

1. Verfahren zum Reinigen einer wässrigen Lösung, die zumindest Ethanol, Acetaldehyd und Diethylacetal umfasst, umfassend:
- einen Schritt A) zur Flüssig-Flüssig-Extraktion im Gegenstrom, der einen Extraktionsabschnitt umfasst, dem am Kopf die wässrige Lösung, gemischt mit mindestens einer Fraktion des Raffinats Wasser/Ethanol/Acetaldehyd aus Schritt B) zur Reextraktion, wobei diese Mischung das Einsatzmaterial für den Extraktionsabschnitt von Schritt A) darstellt, und am Boden ein Extraktionslösungsmittel zugeführt wird, und in dem am Kopf ein Extrakt und am Boden ein gereinigtes Einsatzmaterial erzeugt wird, betrieben bei einer Temperatur zwischen 10 und 40°C und bei einem Druck zwischen 0,1 und 0,5 MPa mit einem Verhältnis Massedurchsatz der kontinuierlichen Phase/Massedurchsatz der dispersen Phase von unter 70;
- einen Schritt B) zur Flüssig-Flüssig-Reextraktion im Gegenstrom, der einen Reextraktionsabschnitt umfasst, dem am Kopf eine wässrige Säurelösung, deren pH-Wert zwischen 0,5 und 5 liegt, und am Boden das Extrakt aus Schritt A) zugeführt wird und in dem am Kopf ein Extrakt und am Boden ein Raffinat Wasser/Ethanol/Acetaldehyd erzeugt wird, betrieben bei einer Temperatur zwischen 10 und 90°C und bei einem Druck zwischen 0,1 und 0,5 MPa, einer Verweilzeit zwischen 0,5 und 6h, mit einem Verhältnis Massedurchsatz der kontinuierlichen Phase/Massedurchsatz der dispersen Phase von unter 70.

2. Verfahren nach Anspruch 1, wobei der Gehalt an Ethanol in der wässrigen Lösung, die Ethanol, Acetaldehyd und Diethylacetal umfasst, zwischen 40 Gewichts-% und 70 Gewichts-% beträgt, der Gehalt an Acetaldehyd zwischen 1 und 30 Gewichts-% beträgt, der Gehalt an Verunreinigungen zwischen 1 und 20 Gewichts-% beträgt und der Gehalt an Diethylacetal zwischen 1 und 20 Gewichts-% beträgt.

3. Verfahren nach einem der vorhergehenden Ansprüche, wobei das Extraktionslösungsmittel, das Schritt A) zugeführt wird, eine Mischung aus Kohlenwasserstoffen mit zwischen 6 und 40 Kohlenstoffatomen ist.

4. Verfahren nach einem der vorhergehenden Ansprüche, wobei das Verhältnis Massedurchsatz der kontinuierlichen Phase/Massedurchsatz der dispersen Phase in dem Extraktionsabschnitt von Schritt A) unter 3 liegt.

5. Verfahren nach einem der vorhergehenden Ansprüche, wobei die wässrige Säurelösung, die Schritt B) zugeführt wird, einen pH-Wert zwischen 2 und 4 aufweist.

6. Verfahren nach einem der vorhergehenden Ansprüche, wobei die Verweilzeit t in dem Reextraktionsabschnitt von Schritt B) zur Reextraktion, ausgedrückt in Stunden, und der pH-Wert der wässrigen Säurelösung, die Schritt B) zugeführt wird, gemeinsam derart geregelt werden, dass pH-log₁₀(t/t₀) zwischen 1 und 4 liegt, wobei t₀ für eine Bezugszeit von 1h steht.

7. Verfahren nach einem der vorhergehenden Ansprüche, wobei die wässrige Säurelösung, die Schritt B) zugeführt wird, weniger als 2 Gewichts-% der Gesamtmenge (Ethanol+Acetaldehyd) enthält.

8. Verfahren nach einem der vorhergehenden Ansprüche, wobei die wässrige Säurelösung, die Schritt B) zugeführt wird, weniger als 1 Gewichts-% der Gesamtmenge (Ethanol+Acetaldehyd) enthält.

9. Verfahren nach einem der vorhergehenden Ansprüche, wobei die wässrige Säurelösung, die Schritt B) zugeführt wird, weder Ethanol noch Acetaldehyd enthält.

10. Verfahren nach einem der vorhergehenden Ansprüche, wobei die organische Phase die kontinuierliche Phase des Extraktionsabschnitts von Schritt A) darstellt und die wässrige Phase die kontinuierliche Phase des Reextraktionsabschnitts von Schritt B) darstellt.

## Claims

1. Process for the purification of an aqueous solution comprising at least ethanol, acetaldehyde and diethyl acetal, comprising
- a stage A) of countercurrentwise liquid-liquid extraction comprising an extraction section fed at the top with said aqueous solution as a mixture with at least a fraction of the water/ethanol/acetaldehyde raffinate resulting from the re-extraction stage B), this mixture constituting the feedstock of said extraction section of said stage A), and at the bottom with an extraction solvent, and producing, at the top, an extract and, at the bottom, a purified feedstock, carried out at a temperature of between 10 and 40°C and at a pressure of between 0.1 and 0.5 MPa with a flow rate by weight of continuous phase/flow rate by weight of dispersed phase ratio of less than 70;
- a stage B) of countercurrentwise liquid-liquid re-extraction comprising a re-extraction section fed at the top with an aqueous acidic solution, the pH of which is between 0.5 and 5, and at the bottom with the extract resulting from stage A), and producing, at the top, an extract and, at the bottom, a water/ethanol/acetaldehyde raffinate, carried out at a temperature of between 10 and 90°C and at a pressure of between 0.1 and 0.5 MPa, a residence time of between 0.5 and 6 h, with a flow rate by weight of continuous phase/flow rate by weight of dispersed phase ratio of less than 70.

2. Process according to Claim 1, in which the content of ethanol in said aqueous solution comprising ethanol, acetaldehyde and diethyl acetal is between 40 and 70 weight%, the content of acetaldehyde is between 1 and 30 weight%, the content of impurities is between 1 and 20 weight% and the content of diethyl acetal is between 1 and 20 weight%.

3. Process according to either of the preceding claims, in which said extraction solvent which feeds stage A) is a mixture of hydrocarbons having between 6 and 40 carbon atoms.

4. Process according to one of the preceding claims, in which the flow rate by weight of continuous phase/flow rate by weight of dispersed phase ratio in said extraction section of said stage A) is less than 3.

5. Process according to one of the preceding claims, in which said aqueous acidic solution which feeds stage B) has a pH of between 2 and 4.

6. Process according to one of the preceding claims, in which the residence time t in said re-extraction section of said re-extraction stage B), expressed in hours, and the pH of the aqueous acidic solution feeding said stage B) are adjusted jointly so that pH-log₁₀ (t/t₀) is between 1 and 4, t₀ representing a reference time equal to 1 h.

7. Process according to one of the preceding claims, in which said aqueous acidic solution which feeds stage B) contains less than 2 weight% of the ethanol + acetaldehyde total.

8. Process according to one of the preceding claims, in which said aqueous acidic solution which feeds stage B) contains less than 1 weight% of the ethanol + acetaldehyde total.

9. Process according to one of the preceding claims, in which said aqueous acidic solution which feeds stage B) does not contain either ethanol or acetaldehyde.

10. Process according to one of the preceding claims, in which the organic phase constitutes the continuous phase of said extraction section of said stage A) and the aqueous phase constitutes the continuous phase of said re-extraction section of said stage B) .
